(19) Europäisches Patentamt
European Patent Office
Office européen des brevets

(11) EP 2 196 483 B1

(12) **EUROPEAN PATENT SPECIFICATION**

(45) Date of publication and mention of the grant of the patent:
**24.10.2012 Bulletin 2012/43**

(21) Application number: **08834086.4**

(22) Date of filing: **22.09.2008**

(51) Int Cl.:
***C08F 297/08*** *(2006.01)* ***A61M 5/178*** *(2006.01)*
***A61M 5/28*** *(2006.01)*

(86) International application number:
**PCT/JP2008/067074**

(87) International publication number:
**WO 2009/041381 (02.04.2009 Gazette 2009/14)**

(54) **POLYPROPYLENE RESIN FOR SYRINGE, SYRINGE OBTAINED BY USING THE SAME AS RAW MATERIAL, AND PRE-FILLED SYRINGE PREPARATION**

POLYPROPYLENHARZ FÜR SPRITZE, DURCH VERWENDUNG DAVON ALS AUSGANGSSTOFF ERHALTENE SPRITZE UND HERSTELLUNG EINER VORGEFÜLLTEN SPRITZE

RÉSINE DE POLYPROPYLÈNE POUR SERINGUE, SERINGUE OBTENUE EN L'UTILISANT COMME MATIÈRE PREMIÈRE ET PRÉPARATION DE SERINGUE PRÉ-REMPLIE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **28.09.2007 JP 2007254735**

(43) Date of publication of application:
**16.06.2010 Bulletin 2010/24**

(73) Proprietors:
- **Mitsui Chemicals, Inc.**
  **Tokyo 105-7117 (JP)**
- **Prime Polymer Co., Ltd.**
  **Tokyo 105-7117 (JP)**

(72) Inventors:
- **ARAI, Takashi**
  **Sodegaura-shi**
  **Chiba 299-0265 (JP)**
- **UEKITA, Hiroyuki**
  **Sodegaura-shi**
  **Chiba 299-0265 (JP)**
- **ITAKURA, Keita**
  **Sodegaura-shi**
  **Chiba 299-0265 (JP)**
- **HASHIZUME, Satoshi**
  **Takaishi-shi**
  **Osaka 592-8501 (JP)**

(74) Representative: **Raynor, Stuart Andrew**
**J A Kemp**
**14 South Square**
**Gray's Inn**
**London WC1R 5JJ (GB)**

(56) References cited:
**WO-A1-2006/068308**
**WO-A1-2007/116709**
**JP-A- 2004 344 423**
**JP-A- 2006 212 148**
**JP-A- 2006 213 800**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 196 483 B1

Printed by Jouve, 75001 PARIS (FR)

## Description

### Technical Field

The present invention relates to a polypropylene resin for syringe, a syringe in which a tubular part (barrel) produced by injection molding of the resin is equipped with a piston (plunger), and a prefilled syringe preparation comprising the syringe filled with a drug solution.

### Background Art

In recent years, in order to decrease problems caused by, for example, wrong drug administration treatment, prefilled syringe preparations filled with drug solution have attracted attention.

In general, the tubular part (barrel) of a prefilled syringe filled with a drug solution is produced from glass or cyclic polyolefin as a raw material and is not widespread in the market due to the high price thereof. On the other hand, polypropylene resins are excellent in chemical properties, physical properties, and molding processability and are also inexpensive, and are therefore being investigated as raw materials for the syringe. However, crack of a syringe and elution of a low molecular weight substance due to a decrease in shock resistance that are caused by interaction with the content liquid and, in particular, scratches formed when a molded syringe is removed from a core molding die are big problems. Therefore, it is the current situation that syringes cannot be stably produced.

As representative of so-called polypropylene resins that have conventionally used, for example, in Examples in Patent Documents 1 and 2, propylene-based random copolymers are used. However, these copolymers are insufficient in strength. In addition, syringes are required to be transparent for visually observing the presence of insoluble fine particles therein. However, the strength is decreased to an insufficient level by controlling the composition of the polypropylene resin for maintaining the transparency. Specifically, in Patent Document 3, a metallocene catalyst system propylene-based random copolymer is applied to an injection-molded product. The method disclosed in the document can provide an injection-molded product excellent in rigidity and transparency, but the injection-molded product has a problem of inferior shock resistance.

As described above, syringes for medical purposes have problems of mechanical strength, transparency, scratches on syringes when they are removed from core molding dies in injection molding, elution of low molecular weight substances, long-run productivity and shock resistance. In addition, there is a problem of sanitary property. There is no polypropylene resin that can solve all of these problems and can be used as a raw material of syringes for medical purposes. Therefore, it is difficult to produce a prefilled syringe filled with a drug solution which solved all of these problems.

If these problems can be solved by using an inexpensive polypropylene resin, it can contribute to prevalence of prefilled syringe preparations filled with drug solutions, can achieve a reduction in medical cost for elderly patients, and thus it can significantly contribute to the society.

Patent Document 1: Japanese Patent No. 3195434
Patent Document 2: Japanese Patent No. 2528443
Patent Document 3: Japanese Patent Application Laid-Open No. H06-192332

### Disclosure of Invention

### Problems to be Solved by the Invention

The present invention is to solve the problems accompanied with the conventional technologies described above and an object of the present invention is to provide a polypropylene resin for syringe that can be used as a raw material for a syringe that is excellent in sanitary property, heat resistance and transparency and hardly elutes low molecular weight substance and that can prevent formation of foam during molding, achieve long-run moldability, and be prevented from scratches when removed from a core molding die in injection molding at satisfactory levels that have not been achieved conventionally.

Another object of the present invention is to provide further safety in medical practice by providing a syringe having satisfactory shock resistance at low temperature which has been conventionally difficult to achieve, and providing an inexpensive prefilled syringe preparation filled with various drug solution having a pH of 5.0 to 9.0, in which shock resistance according to ESCR (a term referring to resistance against a phenomenon that the strength and the shock resistance of a material are decreased by a drug, and also called environmental stress cracking resistance) and drug stability are also considered.

Means for Solving the Problems

The present inventors have conducted intensive studies for solving the above-mentioned problems and, as a result, have found the fact that a polypropylene resin comprising an ethylene-propylene block copolymer satisfying specific requirements can solve the above-mentioned problems. Thus, the present invention has been accomplished.
Accordingly, the essential of the present invention is as follows:
[1] A polypropylene resin for syringe, comprising an ethylene-propylene block copolymer (A)
having a melt flow rate (ASTM D 1238, 230°C, 2.16 kg load) of 10 to 60 g/10 min,
being constituted of 85 to 97% by weight of a room temperature n-decane-insoluble portion ($D_{insol}$) and 3 to 15% by weight of a room-temperature n-decane-soluble portion ($D_{sol}$) (the total amount of the $D_{insol}$ and the $D_{sol}$ is 100% by weight), wherein
the $D_{insol}$ satisfies the following requirements (1) to (3), and the $D_{sol}$ satisfies the following requirements (5) to (7):

(1) the melting point of the $D_{insol}$ is 150 to 165°C,
(2) the molecular weight distribution (Mw/Mn) of the $D_{insol}$ determined by GPC is 1.0 to 3.5,
(3) the content of the unit derived from ethylene in the $D_{insol}$ is 0 to 13% by mole,
(5) the molecular weight distribution (Mw/Mn) of the $D_{sol}$ determined by GPC is 1.0 to 3.5,
(6) the limiting viscosity [η] of the $D_{sol}$ in decalin at 135°C is 1.5 to 4 dl/g, and
(7) the content of the unit derived from ethylene in the $D_{sol}$ is 15 to 25% by mole.

[2] The polypropylene resin for syringe according to [1], wherein the $D_{insol}$ further satisfies the following requirement (4):
(4) the total of 2,1-insertion and 1,3-insertion propylene units in the $D_{insol}$ is 0.2% by mole or less.
[3] The polypropylene resin for syringe according to [1] or [2], wherein the $D_{insol}$ further satisfies the following requirement (1'):
(1') the melting point of the $D_{insol}$ is 150 to 160°C.
[4] The polypropylene resin for syringe according to any one of [1] to [3], wherein the $D_{insol}$ further satisfies the following requirement (3'):
(3') the content of the unit derived from ethylene in the $D_{insol}$ is 0 to 4% by mole;
[5] A syringe in which a tubular part (barrel) obtained by injection molding of the polypropylene resin for syringe according to any one of [1] to [4] is equipped with a piston (plunger).

[6] A prefilled syringe preparation comprising the syringe according to [5] filled with a drug solution having a pH of 5.0 to 9.0.

Advantages of the Invention

According to the present invention, the following three are provided:

(1) a polypropylene resin for syringe that can be used as a raw material for a syringe that is excellent in sanitary property, heat resistance and transparency and hardly elutes low molecular weight substance and that can prevent formation of foam during molding, achieve long-run moldability and being prevented from scratches when removed from a core molding die in injection molding at satisfactory levels that have not been achieved conventionally;
(2) a syringe in which a tubular part (barrel) produced from the resin as the raw material is equipped with a piston (plunger);
(3) an inexpensive prefilled syringe preparation which comprises the syringe filled with various drug solution having a pH of 5.0 to 9.0 and achieved shock resistance according to ESCR and drug stability.

Furthermore, even if the prefilled syringe preparation is filled with a drug solution followed by sterilization by heat, a reduction in transparency and elution of low molecular weight substance hardly occur, and therefore the prefilled syringe preparation is expected to rapidly diffuse in the market.

Best Modes for Carrying Out the Invention

The polypropylene resin according to the present invention and the use thereof will be specifically described below.

(1) Polypropylene resin for syringe

The polypropylene resin for syringe according to the present invention comprises an ethylene-propylene block copolymer (A) having a melt flow rate (ASTM D 1238, 230°C, 2.16 kg load) of 10 to 60 g/10 min and is constituted of 85 to 97% by weight of a room temperature n-decane-insoluble portion ($D_{insol}$) and 3 to 15% by weight of a room-temperature n-decane-soluble portion ($D_{sol}$) (the total amount of the $D_{insol}$ and the $D_{sol}$ is 100% by weight). The $D_{insol}$ satisfies the following requirements (1) to (3), and the $D_{sol}$ satisfies the following requirements (5) to (7).

(1) the melting point of the $D_{insol}$ is 150 to 165°C
(2) the molecular weight distribution (Mw/Mn) of the $D_{insol}$ determined by GPC is 1.0 to 3.5
(3) the content of the unit derived from ethylene in the $D_{insol}$ is 0 to 13% by mole
(5) the molecular weight distribution (Mw/Mn) of the $D_{sol}$ determined by GPC is 1.0 to 3.5
(6) the limiting viscosity [η] of the $D_{sol}$ in decalin at 135°C is 1.5 to 4 dl/g
(7) the content of the unit derived from ethylene in the $D_{sol}$ is 15 to 25% by mole

<Ethylene-propylene block copolymer (A)>

The ethylene-propylene block copolymer (A) constituting the polypropylene resin of the present invention is produced preferably in the presence of a metallocene catalyst system
by producing a propylene-based (co)polymer by homopolymerization of propylene or (co)polymerization of propylene and a small amount of ethylene in a first polymerization step and subsequently producing propylene-ethylene random copolymer rubber in a second polymerization step.

The ethylene-propylene block copolymer (A) has a melt flow rate of 10 to 60 g/10 min and is constituted of 85 to 97% by weight, preferably 88 to 95% by weight and more preferably 90 to 94% by weight of a room-temperature n-decane-insoluble portion ($D_{insol}$) whose main component is the propylene-based (co)polymer produced in the first polymerization step and 3 to 15% by weight, preferably 5 to 12% by weight and more preferably 6 to 10% by weight of a room-temperature n-decane-soluble portion ($D_{sol}$) whose main component is the propylene-ethylene random copolymer rubber produced in the second polymerization step. The total amount of the $D_{insol}$ and the $D_{sol}$ is 100% by weight.

Herein, the ethylene-propylene block copolymer (A) is preferably prepared by polymerization by a metallocene catalyst system.

The ethylene-propylene block copolymer (A) constituting the polypropylene resin for syringe according to the present invention has an MFR in the range of 10 to 60 g/10 min. In particular, when the molecular weight distribution of the polymer is 2.0 or more and less than 2.5, the MFR is preferably 30 to 60 g/10 min and more preferably 40 to 60 g/10 min. When the molecular weight distribution is 2.5 or more and 3.5 or less, the MFR is preferably 10 to 40 g/10 min, more preferably 20 to 35 g/10 min and further preferably 20 to 30 g/10 min.

Since the fluidity of the polypropylene resin for syringe of the present invention is favorable when the MFR is within the above-mentioned range, the syringe can be produced by multi-cavity injection molding, and shock resistance can be maintained. When the MFR is not within the range, that is, when the MFR is lower than 10, the syringe cannot be produced by multi-cavity injection molding, and therefore the actual productivity is low. When the MFR is higher than 60, the molecular weight of the ethylene-propylene block copolymer (A) is too low to maintain the shock resistance, and also low molecular weight substance may elute in the case of a syringe produced by injection molding.

In addition, in the ethylene-propylene block copolymer (A) of the present invention, the $D_{insol}$ satisfies the following requirements (1) to (3), and the $D_{sol}$ satisfies the following requirements (5) to (7).

(1) the melting point of the $D_{insol}$ is 150 to 165°C
(2) the molecular weight distribution (Mw/Mn) of the $D_{insol}$ determined from GPC is 1.0 to 3.5
(3) the content of the unit derived from ethylene in the $D_{insol}$ is 0 to 13% by mole
(5) the molecular weight distribution (Mw/Mn) of the $D_{sol}$ determined by GPC is 1.0 to 3.5
(6) the limiting viscosity [η] of the $D_{sol}$ in decalin at 135°C is 1.5 to 4 dl/g
(7) the content of the unit derived from ethylene in the $D_{sol}$ is 15 to 25% by mole

Furthermore, in the ethylene-propylene block copolymer (A), the $D_{insol}$ preferably satisfies the following requirement (4) in addition to the above-mentioned requirements.

(4) the total amount of the 2,1-insertion and 1,3-insertion propylene units in the $D_{insol}$ is 0.2% by mole or less.

The above-mentioned requirements (1) to (7) will be described in detail below.

Requirement (1)

The room-temperature n-decane-insoluble portion ($D_{insol}$) of the ethylene-propylene block copolymer (A) constituting the polypropylene resin of the present invention has a melting point of 150 to 165°C and preferably 150 to 160°C.

A first advantage by controlling the melting point within this range is an improvement in transparency of the syringe that is obtained by injection molding of the polypropylene resin for syringe according to the present invention. By controlling the melting point within the above-mentioned range, a reduction in transmissivity after water vapor sterilization of the syringe can also be inhibited.

A second advantage is an enhancement in shock resistance of the syringe. Since a syringe is formed by molding the tubular part (barrel) by melting a polypropylene resin, a distortion and a weld tend to remain, and also flow orientation crystallization (solidification with crystallization in flowing) of polypropylene further causes a distortion. This has a problem that breakage tends to occur when shock is applied. The present inventors have newly found that by controlling the melting point within the above-mentioned range, in particular, the orientation crystallization of polypropylene can be significantly reduced and the shock resistance can be enhanced.

A third advantage is that the low-temperature shock resistance of the syringe is improved by the interaction between the n-decane-insoluble portion ($D_{insol}$) having a melting point within the above-mentioned range and the room-temperature n-decane-soluble portion ($D_{sol}$).

Requirement (2)

The molecular weight distribution (Mw/Mn) determined by GPC of the room-temperature n-decane-insoluble portion ($D_{insol}$) of the ethylene-propylene block copolymer (A) constituting the polypropylene resin for syringe of the present invention is 1.0 to 3.5, preferably 1.5 to 3.2 and more preferably 1.8 to 3.0. Since the amount of the low molecular weight component in the ethylene-propylene block copolymer (A) is very small by controlling the molecular weight distribution within a narrow range as described above, elution of the low molecular weight substance hardly occurs in the syringe produced using the polypropylene resin according to the present invention.

Furthermore, regarding the room-temperature n-decane-insoluble portion ($D_{insol}$) constituting a part of the ethylene-propylene block copolymer (A), the molecular weight distribution (Mw/Mn) determined by GPC can be narrowed as described above because of the use of a metallocene catalyst system as the catalyst. When the Mw/Mn is larger than 3.5, the amount of the low molecular weight component is increased, resulting in occurrence of bleed-out to increase the amount of elution, which may reduce the transparency of the syringe after water vapor sterilization.

Requirement (3)

The content of the unit derived from ethylene in the room-temperature n-decane-insoluble portion ($D_{insol}$) of the ethylene-propylene block copolymer (A) constituting the polypropylene resin for syringe of the present invention is 0 to 13% by mole, preferably 0 to 10% by mole, more preferably 0 to 8% by mole, further preferably 0 to 4% by mole and most preferably 0 to 2% by mole. A decrease in the content of the unit derived from ethylene in the $D_{insol}$ heightens the melting point (Tm) of the ethylene-propylene block copolymer (A), which improves heat resistance. On the other hand, when the content of the unit derived from ethylene in the $D_{insol}$ is higher than 13% by mole, the melting point of the ethylene-propylene block copolymer (A) is low, which tends to cause problems such as a decrease in rigidity of the syringe under high temperature.

Requirement (4)

The ethylene-propylene block copolymer (A) constituting the polypropylene resin for syringe of the present invention preferably satisfies the following requirement (4). (4) the total amount of 2,1-insertion and 1,3-insertion propylene units in the room-temperature n-decane-insoluble, portion ($D_{insol}$) is 0.2% by mole or less

More preferably, the total amount of 2,1-insertion and 1,3-insertion propylene units in the $D_{insol}$ of the ethylene-propylene block copolymer (A) is 0.1% by mole or less. When the total amount of 2,1-insertion and 1,3-insertion propylene units in the $D_{insol}$ is larger than 0.2% by mole, the random copolymerization property between propylene and ethylene is decreased, which, as a result, broaden the composition distribution of the propylene-ethylene copolymer rubber in the room-temperature n-decane-soluble portion ($D_{sol}$). Consequently, the shock resistance of the syringe is decreased, and problems such as a reduction in transparency after water vapor sterilization may be caused.

Requirement (5)

The molecular weight distribution (Mw/Mn) determined by GPC of the room-temperature n-decane-soluble portion ($D_{sol}$) of the ethylene-propylene block copolymer (A) constituting the polypropylene resin for syringe of the present invention is 1.0 to 3.5, preferably 1.2 to 3.0 and more preferably 1.5 to 2.5. Since the molecular weight distribution is narrow as mentioned above, the amount of low molecular weight component in the ethylene-propylene block copolymer (A) is very small. Therefore, in the syringe produced using the polypropylene resin according to the present invention, elution of low molecular weight substance hardly occurs.

Regarding the room-temperature n-decane-soluble portion ($D_{sol}$) of the ethylene-propylene block copolymer (A), the molecular weight distribution (Mw/Mn) determined by GPC can be narrowed as described above because of the use of a metallocene catalyst system as the catalyst. When the Mw/Mn is larger than 3.5, the amount of propylene-ethylene random copolymer rubber with a low molecular weight is increased in the $D_{sol}$, which causes bleed-out to reduce transparency of the syringe after water vapor sterilization and may cause problems such as a decrease in shock resistance of the syringe.

Requirement (6)

The limiting viscosity [η] of the room-temperature n-decane-soluble portion ($D_{sol}$) of the ethylene-propylene block copolymer (A) constituting the polypropylene resin for syringe of the present invention in decalin at 135°C is 1.5 to 4 dl/g, preferably 1.5 to 3.5 dl/g, more preferably 1.8 to 3.5 dl/g and most preferably 2.0 to 3.0 dl/g.

In the production of such a block copolymer, if an existing metallocene catalyst other than the metallocene catalyst system that is suitably used in the present invention is employed, it is very difficult to produce an ethylene-propylene block copolymer in which the limiting viscosity [η] of the $D_{sol}$ is higher than 1.5 dl/g. In particular, it is almost impossible to produce an ethylene-propylene block copolymer in which the limiting viscosity [η] of the $D_{sol}$ is 1.8 dl/g or higher.

When the limiting viscosity [η] of the $D_{sol}$ in decalin at 135°C is higher than 4 dl/g, a slight amount of propylene-ethylene random copolymer rubber having an ultra-high molecular weight or containing a large amount of ethylene is by-produced during the production of the propylene-ethylene random copolymer rubber in the second polymerization step. Since the propylene-ethylene random copolymer rubber by-produced in a slight amount is unevenly present in the ethylene-propylene block copolymer, the syringe produced using such a copolymer as the raw material tends to have lowered shock resistance.

Requirement (7)

The content of the unit derived from ethylene in the n-decane-soluble portion ($D_{sol}$) of the ethylene-propylene block copolymer (A) constituting the polypropylene resin for syringe of the present invention is 15 to 25% by mole, preferably 15 to 22% by mole and more preferably 16 to 20% by mole. When the content of the unit derived from ethylene in the $D_{sol}$ is lower than 15% by mole, the shock resistance of the ethylene-propylene block copolymer (A) may be low. On the other hand, when the content of the unit derived from ethylene in the $D_{sol}$ is higher than 25% by mole, the syringe may have low transparency.

<Method of producing polypropylene resin for syringe>

Next, a method of producing the polypropylene resin for syringe according to the present invention will be described in detail below.

The ethylene-propylene block copolymer (A) constituting the polypropylene resin for syringe according to the present invention can be prepared in the presence of a specific metallocene-based catalyst for polymerization and a promoter, preferably, by producing a propylene-based (co)polymer by homopolymerization of propylene or (co)polymerization of propylene and a small amount of ethylene in a first polymerization step ([step 1]) and subsequently producing propylene-ethylene random copolymer rubber in a second polymerization step ([step 2]) by copolymerization of propylene and ethylene in an amount larger than that in the first step.

The metallocene catalyst for polymerization is preferably a metallocene catalyst that can cause a polymerization with a stereoregularity such as an isotactic or syndiotactic structure, and examples thereof are catalysts shown below which are described in WO01/27124 pamphlet:

[Formula 1]

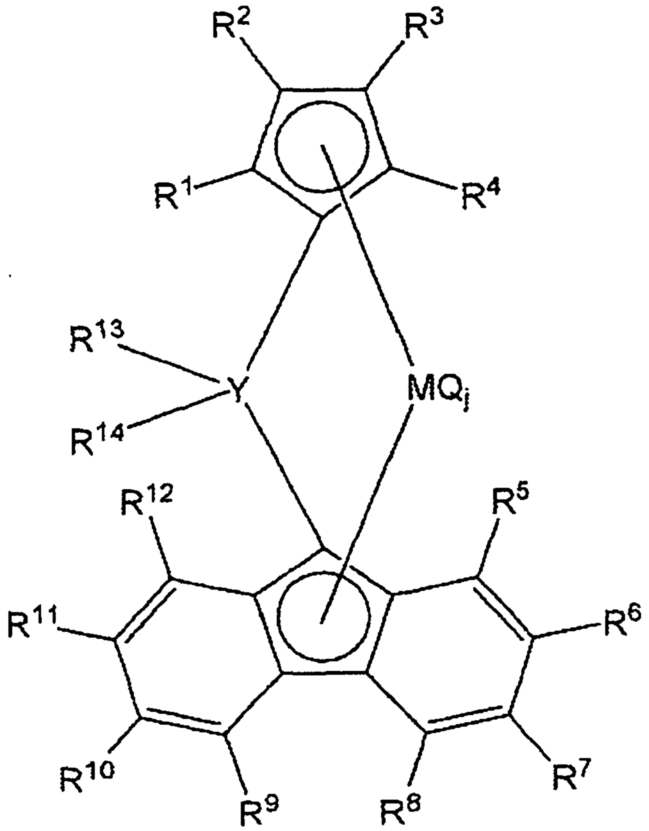

• • • [I]

In the above general Formula [I], $R^1$, $R^2$, $R^3$, $R^4$, $R^5$, $R^6$, $R^7$, $R^8$, $R^9$, $R^{10}$, $R^{11}$, $R^{12}$, $R^{13}$ and $R^{14}$ may be the same or different and are selected from hydrogen atoms, hydrocarbon groups and silicon-containing groups. Examples of the hydrocarbon groups include linear hydrocarbon groups such as a methyl group, an ethyl group, a n-propyl group, an allyl group, a n-butyl group, a n-pentyl group, a n-hexyl group, a n-heptyl group, a n-octyl group, a n-nonyl group and a n-decanyl group; branched hydrocarbon groups such as an isopropyl group, a tert-butyl group, an amyl group, a 3-methylpentyl group, a 1,1-diethylpropyl group, a 1,1-dimethylbutyl group, a 1-methyl-1-propylbutyl group, a 1,1-propylbutyl group, a 1,1-dimethyl-2-methylpropyl group and a 1-methyl-1-isopropyl-2-methylpropyl group; saturated cyclic hydrocarbon groups such as a cyclopentyl group, a cyclohexyl group, a cycloheptyl group, a cyclooctyl group, a norbornyl group and an adamantyl group; unsaturated cyclic hydrocarbon groups such as a phenyl group, a tolyl group, a naphthyl group, a biphenyl group, a phenanthryl group and an anthracenyl group; saturated hydrocarbon groups substituted with an unsaturated cyclic hydrocarbon group such as a benzyl group, a cumyl group, a 1,1-diphenylethyl group and a triphenylmethyl group; and hetero atom-containing hydrocarbon groups such as a methoxy group, an ethoxy group, a phenoxy group, a furyl group, a N-methylamino group, a N,N-dimethylamino group, a N-phenylamino group, a pyryl group and a thienyl group. Examples of the silicon-containing groups include a trimethylsilyl group, a triethylsilyl group, a dimethylphenylsilyl group, a diphenylmethylsilyl group and a triphenylsilyl group.

Furthermore, in the general Formula [I], each substituent of $R^5$ to $R^{12}$ may form a ring together with an adjacent substituent. Examples of such a substituted fluorenyl group include a benzofluorenyl group, a dibenzofluorenyl group, an octahydrodibenzofluorenyl group, an octamethyloctahydrodibenzofluorenyl group and an octamethyltetrahydrodicyclopentafluorenyl group.

In the general Formula [I], $R^1$, $R^2$, $R^3$ and $R^4$ bonding to the cyclopentadienyl ring are each preferably a hydrogen atom or a hydrocarbon group having 1 to 20 carbon atoms. Examples of the hydrocarbon group having 1 to 20 carbon atoms include the hydrocarbon groups mentioned above. More preferably, $R^3$ is a hydrocarbon group having 1 to 20 carbon atoms.

In the general Formula [I], $R^5$ to $R^{12}$ bonding to the fluorene ring are each preferably a hydrocarbon group having 1 to 20 carbon atoms. Examples of the hydrocarbon group having 1 to 20 carbon atoms include the hydrocarbon groups mentioned above. Each substituent, $R^5$ to $R^{12}$, may form a ring together with an adjacent substituent.

In the general Formula [I], Y bridging the cyclopentadienyl ring and the fluorenyl ring is preferably a group XIV element of the periodic table, more preferably carbon, silicon, or germanium and further preferably a carbon atom. $R^{13}$ and $R^{14}$ bonding to the Y are each preferably a hydrocarbon group having 1 to 20 carbon atoms. They may be the same

or different from each other and may form a ring together. Examples of the hydrocarbon group having 1 to 20 carbon atoms include the hydrocarbon groups mentioned above. More preferably, $R^{14}$ is an aryl group having 6 to 20 carbon atoms.

Examples of the aryl group include the above-mentioned unsaturated cyclic hydrocarbon groups, saturated hydrocarbon groups substituted with an unsaturated cyclic hydrocarbon group and hetero atom-containing unsaturated cyclic hydrocarbon groups. $R^{13}$ and $R^{14}$ may be the same or different from each other and may form a ring together. Preferred examples of the substituent include a fluorenylidene group, a 10-hydroanthracenylidene group and a dibenzocycloheptadienylidene group.

In the metallocene compound represented by the above general Formula [I], a substituent selected from $R^1$, $R^4$, $R^5$ and $R^{12}$ and $R^{13}$ or $R^{14}$ in bridging part may bond to each other to form a ring.

In the general Formula [I], M is preferably a group IV transition metal of the periodic table and more preferably Ti, Zr or Hf.

Furthermore, each Q, which may be the same or different, is selected from halogen atoms, hydrocarbon groups, anionic ligands and neutral ligands that can be coordinated with lone pair. J is an integer of 1 to 4. When j is 2 or higher, Qs may be the same or different from each other. Specific examples of the halogen atoms include a fluorine atom, a chlorine atom, a bromine atom and an iodine atom. Specific examples of the hydrocarbon groups include those mentioned above. Specific examples of the anionic ligands include alkoxy groups such as methoxy, tert-butoxy and phenoxy; carboxylate groups such as acetate and benzoate; and sulfonate groups such as mesylate and tosylate. Specific examples of the neutral ligands that can be coordinated with lone pair include organic phosphorus compounds such as trimethylphosphine, triethylphosphine, triphenylphosphine and diphenylmethylphosphine; and ethers such as tetrahydrofuran, diethyl ether, dioxane and 1,2-dimethoxyethane. At least one Q is preferably a halogen atom or an alkyl group.

Preferred examples of the bridged metallocene compounds include isopropyl(3-t-butyl-5-methylcyclopentadienyl)(3,6-dit-butylfluorenyl)zirconium dichloride, diphenylmethylene(3-tert-butyl-5-methyl-cyclopentadienyl)(fluorenyl) zirconium dichloride, diphenylmethylene(3-tert-butyl-5-methylcyclopentadienyl)(2,7-ditert-butylfluorenyl)zirconium dichloride, diphenylmethylene(3-tert-butyl-5-methylcyclopentadienyl)(3,6-ditert-butylfluorenyl)zirconium dichloride, (methyl)(phenyl)methylene(3-tert-butyl-5-methylcyclopentadienyl) (octamethyloctahydrobenzofluorenyl)zirconium dichloride and [3-(1',1',4',4',7',7',10',10'-octamethyloctahydrodibenzo[b,h]fluorenyl)(1,1,3-trimethyl-5-tert-butyl-1,2,3,3a-tetrahydropentalene)]zirconium dichloride (refer to the following Formula [II]).

[Formula 2]

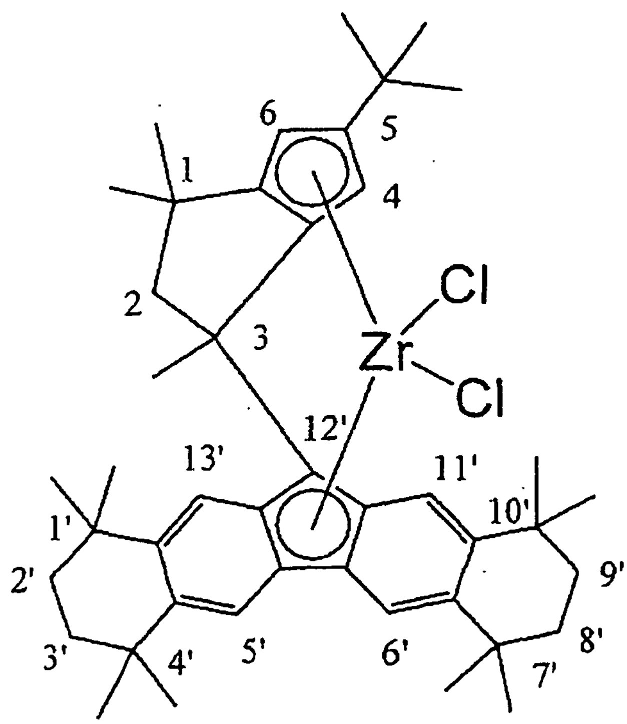

• • • [II]

In the metallocene catalyst used in the present invention, the promoter that is used in conjunction with a group IV transition metal compound represented by the general Formula [I] is comprises at least one compound selected from organic metal compounds, organic aluminum oxy compounds and compounds that react with transition metal compounds to form ion pairs, and a particle-shaped carrier that is used according to need. As them, the compounds disclosed in the publication by the present applicant (WO01/27124 pamphlet), or disclosed in Japanese Patent Application Laid-Open No. H11-315109 can be used without any limitation.

The ethylene-propylene block copolymer (A) constituting the polypropylene resin according to the present invention can be obtained by sequentially performing two steps ([step 1] and [step 2]) described below by using, for example, a polymerization apparatus in which two ore more reaction apparatuses are connected in series.

([Step 1])

In [step 1], usually, propylene is homopolymerized at a polymerization temperature of 0 to 100°C at a polymerization pressure of ordinary pressure to 5 MPa gauge pressure, or propylene and a small amount of ethylene are copolymerized. In [Step 1], the feed amount of the ethylene is controlled to be zero or small with respect to that of the propylene, so that the propylene-based (co)polymer produced in [step 1] is the main component of $D_{insol}$. The homopolymerization of propylene or the copolymerization of propylene and ethylene may be performed by any of batch, semicontinuous and continuous methods or may be performed by multistage polymerization using a plurality of reactors. Furthermore, [step 1] may be conducted as block polymerization in which the feed amount of ethylene is varied with respect to that of propylene.

([Step 2])

In [step 2], usually, propylene and ethylene are copolymerized at a polymerization temperature of 0 to 100°C at a polymerization pressure of ordinary pressure to 5 MPa gauge pressure. In [step 2], the feed amount of the ethylene with respect to that of the propylene is controlled larger than that in [step 1], so that the propylene-ethylene random copolymer rubber produced in [step 2] is the main component of $D_{sol}$.

By thus doing, the requirements (1) to (4) on the $D_{insol}$ can be satisfied by controlling the polymerization conditions in [step 1], and the requirements (5) to (7) on the $D_{sol}$ can be satisfied by controlling the polymerization conditions in [step 2].

In addition, the physical properties that should be satisfied by the ethylene-propylene block copolymer (A) constituting the polypropylene resin of the present invention are determined mainly depending on the chemical structure of the metallocene catalyst used. Specifically, the molecular weight distribution (Mw/Mn) of the $D_{insol}$ determined by GPC of the requirement (2), the total amount of 2,1-insertion and 1,3-insertion propylene units in the $D_{insol}$ of the requirement (4), and the molecular weight distribution (Mw/Mn) of the $D_{sol}$ determined by GPC required of the requirement (5) can be adjusted so as to satisfy the requirements in the present invention mainly by suitably selecting the metallocene catalysts used in [step 1] and [step 2]. Preferred examples of the metallocene catalyst used in the present invention are as described above.

Furthermore, the content of the unit derived from ethylene in the $D_{insol}$ of the requirement (3) can be adjusted by the feed amount of the ethylene in [step 1] or the like. The limiting viscosity [$\eta$] of the $D_{sol}$ in decalin at 135°C of the requirement (6) can be adjusted by the feed amount of a molecular weight-adjusting agent such as hydrogen in [step 2] or the like. The content of the unit derived from ethylene in the $D_{sol}$ of the requirement (7) can be adjusted by the feed amount of the ethylene in [step 2] or the like. In addition, the composition ratio of the $D_{insol}$ and the $D_{sol}$ and the melt flow rate of the ethylene-propylene block copolymer (A) can be suitably adjusted by controlling the amount ratio of the polymers produced in [step 1] and [step 2].

Furthermore, the ethylene-propylene block copolymer (A) of the present invention may be produced by separately producing the propylene-based (co)polymer in [step 1] by the above-mentioned method and the propylene-ethylene random copolymer rubber in [step 2] by the above-mentioned method, in the presence of a metallocene compound-containing catalyst, and then blending them by physical means.

The polypropylene resin for syringe according to the present invention satisfies the prevention of foam formation during molding, the long-run moldability and the prevention of scratches when removed from a core molding die in injection molding at levels that have not been achieved conventionally. Injection molding of such a resin can provide a syringe that is excellent in sanitary property, heat resistance and transparency and hardly elutes low molecular weight substance.

The polypropylene resin according to the present invention may contain a phosphorus-based antioxidant, an amine-based antioxidant or a hydrochloric acid absorber represented by hydrotalcite, according to need.

The phosphoru-based antioxidant is not particularly limited, and conventionally known phosphorus-based antioxidants can be used. The use of one type of the phosphorus-based antioxidant alone leads to low contamination of the contents by bleeding, and is therefore preferred. Trivalent organic phosphorus compounds are preferred as the phosphorus-based antioxidants, and specific examples thereof include tris(2,4-di-t-butylphenyl)phosphite, 2,2-methylenebis (4,6-di-t-butylphenyl)octylphosphite and bis(2,6-di-t-butyl-4-methylphenyl)pentaerythritol diphosphite.

The phosphorus-based antioxidant is used in an amount of usually 0.03 to 0.3 parts by weight, preferably 0.05 to 0.2 parts by weight and particularly preferably 0.08 to 0.15 parts by weight based on 100 parts by weight of the ethylene-propylene block copolymer (A) constituting the polypropylene resin according to the present invention.

Examples of the amine-based antioxidant include compounds having a piperidine ring, specifically, dimethyl succinate·1-(2-hydroxyethyl)-4-hydroxy-2,2,6,6-tetramethylpiperidine polycondensate, bis(2,2,6,6-tetramethyl-4-piperidyl)sebacate and poly[[6-[1-[(1,1,3,3-tetramethylbutyl)amino]-s-triazin-2,4-diyl][[(2,2,6,6-tetramethyl-4-piperidyl)imino] hexamethylene[(2,2,6,6-tetramethyl-4-piperidyl)imino]].

The amine-based antioxidant is used in an amount of usually 0.01 to 0.3 parts by weight, preferably 0.01 to 0.2 parts by weight, more preferably 0.01 to 0.1 part by weight and particularly preferably 0.03 to 0.06 parts by weight based on 100 parts by weight of the ethylene-propylene block copolymer (A) .

The hydrochloric acid absorber is preferably hydrotalcite, considering the sterilization method, generation of white turbid substance with a drug solution and interaction with a nucleator. However, only in the case of no reactivity with the drug solution, a combination system with hydrotalcite having a significantly reduced amount of metal salts of fatty acid can also be suitably used. The additive amount of the hydrochloric acid absorber is usually 0.02 to 0.20 parts by weight, preferably 0.02 to 0.10 parts by weight and more preferably 0.02 to 0.05 parts by weight based on 100 parts by weight of the ethylene-propylene block copolymer (A).

The polypropylene resin according to the present invention may further contain, according to need, a heat-resistant stabilizer, an antistatic agent, a mold lubricant, a slip agent, a light stabilizer, a UV absorber, a pigment, a dye or the like in the ranges that do not impair the purposes of the present invention.

Furthermore, in order to impart transparency to the polypropylene resin according to the present invention, a nucleator may be added. As the nucleator, general nucleators can be used. For example, ADK STAB NA-11 represented by organic phosphate ester is most common. However, in order to satisfy the ultraviolet absorption spectrum, the standards for plastic containers for aqueous injection products (polyethylene or polypropylene containers for injection products) of "Test Methods for Plastic Containers for Pharmaceutical Products Test" in General Tests of the Japanese Pharmacopoeia, 15th Edition, it is desirable not to add a sorbitol-based nucleator.

The nucleator is used in an amount of usually 0.01 to 0.5 parts by weight, preferably 0.05 to 0.2 parts by weight and more preferably 0.08 to 0.15 parts by weight based on 100 parts by weight of the ethylene-propylene block copolymer (A).

The polypropylene resin for syringe according to the present invention can be prepared by, according to need, adding a nucleator and other additives such as a phosphorus-based antioxidant, an amine-based antioxidant or a hydrochloric acid absorber to the ethylene-propylene block copolymer (A); mixing them with a Henschel mixer, a V-type blender, a tumbler blender, a ribbon blender or the like; and then melt-kneading the mixture using a single screw extruder, a multi-screw extruder, a kneader, a banbury mixer or the like, with such a state that the above-mentioned each components and the additives are uniformly dispersed and mixed.

<u>(2) Syringe</u>

The syringe according to the present invention comprises a tubular part (barrel) obtained by injection molding of the polypropylene resin for syringe according to the present invention which is equipped with a piston (plunger). The injection molding of the polypropylene resin for syringe can be performed by a method that is usually employed. The polypropylene resin for syringe satisfies the prevention of formation of foam during molding, the long-run moldability and prevention of scratches when removed from a core molding die in injection molding at levels that have not been achieved conventionally. Therefore, injection molding using such a resin can provide a syringe that is excellent in sanitary property, heat resistance and transparency, hardly elutes low molecular weight substance and is further improved in low-temperature shock resistance.

Incidentally, any known piston (plunger) can be used without limitation as the piston (plunger).

<u>(3) Prefilled syringe preparation</u>

The drug solution used in the prefilled syringe preparation according to the present invention desirably has a pH of 5.0 to 9.0. As long as the pH of a drug solution is within the above range, no adsorption of the drug solution components to the syringe occurs in water vapor sterilization or long period storage, resulting in no decrease in the effect of the drug solution.

The drug solution is not particularly limited as long as the pH thereof is within the above-mentioned range, but is particularly desirable to be a neutral (having a pH of about 7.0) drug solution represented by, for example, heparin aqueous solution or potassium chloride aqueous solution. Highly volatile preparations and high-concentration alcohol

preparations cause deformation of the syringe by an increase in the internal pressure during sterilization and are therefore excluded from the drug solution regardless of the pH.

The prefilled syringe preparation according to the present invention comprises a syringe in which a tubular part (barrel) obtained by injection molding of the polypropylene resin for syringe according to the present invention is equipped with a piston (plunger) and which is filled with a drug solution having a pH of 5.0 to 9.0. The polypropylene resin for syringe can prevent formation of foam during molding, maintain high cycle productivity and be prevented from scratches in the inner surface of the syringe during long-run injection molding at satisfactory levels that have not been achieved conventionally. Consequently, by injection molding of it, a syringe having rigidity, heat resistance, sanitary property, and, in particular, high transparency that has not been achieved conventionally, being excellent in shock resistance and also hardly eluting low molecular weight substance can be produced.

In addition, since the prefilled syringe preparation of the present invention is sterilized with vapor at 121°C once or twice during the production process, the syringe is required to have high heat resistance not to be deformed by the vapor. Since the syringe of the prefilled syringe preparation of the present invention has high heat resistance, no deformation occurs on the syringe even after sterilization.

As above, the syringe and the prefilled syringe preparation comprising the syringe filled with a drug solution having a pH of 5.0 to 9.0 achieve all the properties with excellent balance, which has not been achieved by conventional polypropylene resins.

Examples

Next, the present invention will now be described with reference to examples, but is not limited to these examples.

In the examples, the physical properties were measured by the following methods.

(1) Melt flow rate (MFR)

The MFR was measured in accordance with ASTM D 1238 at 230°C at a load of 2.16 kg.

(2) Ethylene content

The ethylene content was measured by conducting the measurement by IR.

(3) Melting point (Tm)

The measurement was carried out using a differential scanning calorimeter (DSC, manufactured by Perkin Elmer Inc.). Here, the endothermic peak measured in the third step was defined as the melting point (Tm).

(Measurement condition)

First step: an increase in temperature at a rate of 10°C/min up to 240°C, followed by holding the temperature for 10 min.

Second step: a decrease in temperature at a rate of 10°C/min down to 60°C.

Third step: an increase in temperature at a rate of 10°C/min up to 240°C.

(4) Amount of room-temperature n-decane-soluble portion ($D_{sol}$)

200ml of n-decane was added to 5 g of a sample of the final product (that is, the ethylene-propylene block copolymer (A) constituting the polypropylene resin of the present invention), which were heated at 145°C for 30 minutes for dissolving. The resulting was cooled down to 20°C over about 3 hours and then left for standing for 30 minutes. Then, the precipitate (hereinafter, n-decane-insoluble portion: $D_{insol}$) was separated by filtration. The filtrate was put into acetone with a volume about three times of the filtrate volume of for precipitating the components (precipitate (A)) dissolved in the n-decane. The precipitate (A) and the acetone were separated by filtration, and the precipitate was dried. Incidentally, no residue was observed after concentration to dryness of the filtrate.

The amount of the n-decane-soluble portion was determined by the following equation:

n-decane-soluble portion amount (wt%) = [weight of

$$precipitate\ (A)/sample\ weight] \times 100.$$

(5) Mw/Mn measurement [weight average molecular weight (Mw), number average molecular weight (Mn)]

The measurement was performed using GPC-150C Plus, manufactured by Waters Corp., as follows. TSKgel GMH6-HT and TSKgel GMH6-HTL each having an inner diameter of 7.5 mm and a length of 600 mm were used as the separation columns; the column temperature was 140°C; the mobile phase was composed of o-dichlorobenzene (Wako Pure Chemical Industries, Ltd.) and 0.025% by weight of BHT (Wako Pure Chemical Industries, Ltd.) as an antioxidant and was moved at 1.0 mL/min; the sample concentration was set to 0.1% by weight; the injection amount of the sample was 500 μL; and a differential refractometer was used as the detector. The standard polystyrenes were those manufactured by Tosoh Corp. for those having molecular weight of $Mw < 1000$ and $Mw > 4\times10^6$ and were those manufactured by Pressure Chemical Company for those having molecular weight of $1000 \leq Mw \leq 4\times10^6$ was.

(6) Limiting viscosity [η]

The measurement was performed using a decalin solvent at 135°C. A sample (about 20 mg) was dissolved in 15 mL of decalin, and the specific viscosity ηsp was measured in an oil bath at 135°C. Then, the decalin solution was diluted with 5 mL of a decalin solvent, and the specific viscosity ηsp was measured as in above. The dilution operation was further repeated twice. The value of ηsp/C obtained by extrapolating the concentration (C) to zero was determined as the limiting viscosity.
$$[\eta] = \lim(\eta sp/C)\ \ (C \rightarrow 0).$$

(7) Measurement of amounts of 2,1-insertion and 1,3-insertion propylene units

The amounts of 2,1-insertion and 1,3-insertion propylene units were measured using $^{13}C$-NMR according to the method described in Japanese Patent Application Laid-Open No. H07-145212.

(8) Bending elastic modulus

A bending test was performed in accordance with ASTM D 790, and the bending elastic modulus was calculated from the result.

(9) Heat deformation temperature

The measurement was performed in accordance with ASTM D 648. The unit is °C.

(10) Transparency (visibility) evaluation

Square plates having a length of 12 cm, a width of 11 cm, and a thickness of 1 mm of polypropylene resins of Examples and Comparative Examples were injection molded at a melt temperature of 200°C and a die temperature of 30°C. The syringes were water vapor sterilized at 121°C for one hour in accordance with "Test Methods for Plastic Containers for Pharmaceutical Products" for plastic containers for aqueous injection products (polyethylene or polypropylene containers for injection products) in General Tests of the Japanese Pharmacopoeia, 15th Edition. The parallel optical transmittances (%) of the molded products in pure water were measured before and after sterilization.
In the Japanese Pharmacopoeia standards, a parallel optical transmittance of 55% or more is required, but the values of parallel optical transmittance of products made of the same raw material vary depending on the shape and the thickness of the products. The syrnge is designed to have a thickness of 1 mm to 1.2 mm. It is premised that a syringe with 1 mm thickness is judged as acceptable when the syringe has a parallel optical transmittance of 55%. Given that a syringe has a thickness of 1.2 mm, the parallel optical transmittance judged as acceptable (good) was set at 66% or more, i.e. the value increased by twenty percents as compared to 55% for 1mm, and the parallel optical transmittance of less than 66% was judged as unacceptable (no good).

(11) Evaluation of moldability

The syringe producing cycle time (sec) was determined with a 150-t electric injection molder having a molding die for eight syringe outer cylinders each having a volume of 20 mL and a thickness of 1 mm, and one having a time of 6 sec or longer was determined as (no good). In addition, one having an appearance defect such as flash was determined as also (no good) regarding the moldability. One that did not apply to the both was determined as (good).

(12) Evaluation of existence or nonexistence of deformation by heating

Each of the syringes was filled with a heparin aqueous solution (500 μ/mL) and set with a piston. The syringe was subjected to water vapor sterilization at 121°C for 1 hour in accordance with an elution test of the Japanese Pharmacopoeia. The syringe was visually evaluated for deformation.

(13) Shock resistance

Regarding syringe breakage height, a square weight of 45 g was dropped onto the syringe barrel from various heights to determine the syringe breakage height. The average of breakage heights (n=10) was calculated. A syringe that was broken at a height of 50 cm or less was determined as (no good). The measurement was performed at room temperature (23°C).

Next, raw materials for the syringe used in the examples are shown:

1) Ethylene-propylene block copolymer (A) (the physical properties and the production process will be described in the following production examples),
2) Nucleator (B) Sodium-2,2'-methylenebis(4,6-di-t-butylphenyl)phosphate (manufactured by Adeka Corp., product name: ADK STAB NA-11UY),
3) Phosphorus-based antioxidant (C)
Tris(2,4-di-t-butylphenyl)phosphite (manufactured by Ciba Specialty Chemicals Inc., product name: Irgafos 168),
4) Amine-based antioxidant (D)
Dimethyl succinate·1-(2-hydroxyethyl)-4-hydroxy-2,2,6,6-tetramethyl piperidine polycondensate (manufactured by Ciba Specialty Chemicals Inc., product name: Tinuvin 622LD), and
5) Hydrochloric acid absorber (E)
Hydrotalcite represented by $Mg_4Al_2(OH)_{12}CO_3{\cdot}{_3}H_2O$ (manufactured by Kyowa Chemical Industry, Co., Ltd., product name: DHT-4A).

[Production Example 1]

(1) Production of solid catalyst carrier

300.g of $SiO_2$ (Sunsphere H121, manufactured by AGC SI-Tech Co., Ltd.) was sampled into a 1 L side-arm flask and 800 mL of toluene was put into the flask to make them slurry.

Then, the slurry was transferred to a 5 L four-neck flask, followed by addition of 260 mL of toluene.

Furthermore, 2830 mL of a methylaluminoxane (hereinafter, referred to as MAO)/toluene solution (10% by weight solution, manufactured by Albemarle Corp.) was incorporated into the flask, and the mixture was stirred at room temperature for 30 minutes. The temperature of the solution was raised to 110°C over one hour and a reaction was carried out for 4 hours. After the completion of the reaction, the solution was cooled down to room temperature. After the cooling, the supernatant toluene was extracted and replaced with fresh toluene until the replacement ratio reached 95% to prepare MAO/$SiO_2$/toluen slurry.

(2) Production of solid catalyst component (preparation of metal catalyst component supported by a carrier)

In a glove box, 1.4 g of isopropyl(3-t-butyl-5-methylcyclopentadienyl)(3,6-di-t-butylfluorenyl)zirconium dichloride was weighed into a 5 L four-neck flask. The flask was taken out from the glove box, and 0.5 L of toluene and 2.0 L (140 g as a solid component) of the MAO/$SiO_2$/toluene slurry prepared in the above (1) were added under nitrogen, followed by stirring for 30 minutes for supporting.

The resulting isopropyl(3-t-butyl-5-methylcyclopentadienyl)(3,6-di-t-butylfluorenyl)zirconium dichloride/MAO/$SiO_2$/toluene slurry was subjected to 99% substitution with n-heptane so that the final amount of the slurry was 4.5 L. This operation was carried out at room temperature.

(3) Production of prepolymerization catalyst

In a 200 L autoclave equipped with a stirrer and containing 75 L of heptane, 156 mL of triethylaluminum and 144 g of the solid catalyst component prepared in the above (2) were sequentially charged so that the total amount of heptane was adjusted to 80 L. 1440 g of ethylene was added thereto, followed by a reaction with stirring for 180 minutes while keeping the internal temperature at 15 to 20°C.

After the completion of the polymerization, the solid component was precipitated. The supernatant liquid was removed and washing with heptane was carried out twice. The resulting prepolymerization catalyst was resuspended in purified heptane, and the concentration of the solid catalyst component was adjusted to 1 g/L with heptane. The prepolymerization catalyst contained 10 g of polyethylene per 1 g of the solid catalyst component.

(4) Main polymerization

<u>Main polymerization-1 ([step 1])</u>

To a 58 L circulation-type tubular polymerization reactor equipped with a jacket, propylene at 45 kg/hr, hydrogen at 12.5 NL/hr, the catalyst slurry prepared in the above (3) as a solid catalyst component at 2.5 g/hr and triethylaluminum at 1.7 mL/hr were sequentially fed so that the polymerization was conducted under the conditions that the tubular polymerization reactor was filled with the liquid and no gas phase was present. The temperature of the tubular reactor was 30°C, and the pressure was 2.9 MPa/G.

<u>Main polymerization-2 ([step 1])</u>

The resulting slurry was transferred to a 1000 L vessel polymerization reactor equipped with a stirrer for further polymerization. To the polymerization reactor, propylene was fed at 67 kg/hr, and hydrogen was fed so that the hydrogen concentration in the gas phase was 0.45% by mole. The polymerization was conducted at a polymerization temperature of 70°C and at a pressure of 2.8 MPa/G.

<u>Main polymerization-3 ([step 1])</u>

The resulting slurry was transferred to a 500 L vessel polymerization reactor equipped with a stirrer for further polymerization. To the polymerization reactor, propylene was fed at 11 kg/hr, and hydrogen was fed so that the hydrogen concentration in the gas phase was 0.45% by mole. The polymerization was conducted at a polymerization temperature of 68°C and at a pressure of 2.8 MPa/G.

<u>Main polymerization-4 ([step 1])</u>

The resulting slurry was transferred to a 500 L vessel polymerization reactor equipped with a stirrer for further polymerization. To the polymerization reactor, propylene was fed at 14 kg/hr, and hydrogen was fed so that the hydrogen concentration in the gas phase was 0.45% by mole. The polymerization was conducted at a polymerization temperature of 67°C and at a pressure of 2.7 MPa/G.

<u>Copolymerization ([step 2])</u>

The resulting slurry was transferred to a 500 L vessel polymerization reactor equipped with a stirrer for copolymerization. To the polymerization reactor, propylene was fed at 10 kg/hr, and hydrogen was fed so that the hydrogen concentration in the gas phase was 0.1% by mole. Further, the polymerization was conducted by feeding ethylene to keep a polymerization temperature of 63°C and a pressure of 2.9 MPa/G.

After gasification of the resulting slurry, gas-solid separation was performed to give an ethylene-propylene block copolymer (A1). The obtained ethylene-propylene block copolymer (A1) was vacuum dried at 80°C.

[Production Example 2]

(1) Production of solid catalyst carrier

MAO/$SiO_2$/toluene slurry was prepared in the same manner as in the above (1) in Production Example 1.

(2) Production of solid catalyst component (preparation of metal catalyst component supported by a carrier)

In a glove box, 2.0 g of [3-(1',1',4',4',7',7',10',10'-octamethyloctahydrodibenzo[b,h]fluorenyl)(1,1,3-trimethyl-5-tert-butyl-1,2,3,3a-tetrahydropentalene)]zirconium dichloride synthesized according to the production method described in WO2006/068308 pamphlet was weighed into a 5 L four-neck flask. The flask was taken out from the glove box, and 0.46 L of toluene and 1.4 L of the MAO/$SiO_2$/toluene slurry prepared in the above (1) were added under nitrogen, followed by stirring for 30 minutes for supporting.

The resulting [3-(1',1',4',4',7',7',10',10'-octamethyloctahydrodibenzo[b,h]fluorenyl)(1,1,3-trimethyl-5-tert-butyl-1,2,3,3a-tetrahydropentalene)]zirconium dichloride/MAO/$SiO_2$/toluene slurry was subjected to 99% substitution with n-heptane so that the final amount of the slurry was 4.5 L. This operation was carried out at room temperature.

(3) Production of prepolymerization catalyst

Into a 200 L autoclave equipped with a stirrer, 202 g of the solid catalyst component prepared in the above (2), 109 mL of triethylaluminum and 100 L of heptane were incorporated. 2020 g of ethylene was incorporated thereto, followed by a reaction with stirring for 180 minutes while keeping the internal temperature at 15 to 20°C.

After the completion of the polymerization, the solid component was precipitated. The supernatant liquid was removed and washing with heptane was carried out twice. The resulting prepolymerization catalyst was resuspended in purified heptane, and the concentration of the solid catalyst component was adjusted to 2 g/L with heptane. The prepolymerization catalyst contained 10 g of polyethylene per 1 g of the solid catalyst component.

(4) Main polymerization

Main polymerization-1 ([step 1])

To a 58 L circulation-type tubular polymerization reactor equipped with a jacket, propylene at 40 kg/hr, hydrogen at 5 NL/hr, the catalyst slurry prepared in the above (3), as a solid catalyst component at 1.6 g/hr and triethylaluminum at 1.0 g/hr were sequentially fed so that the polymerization was conducted under the conditions that the tubular polymerization reactor was filled with the liquid and no gas phase was present. The temperature of the tubular reactor was 30°C, and the pressure was 3.2 MPa/G.

Main polymerization-2 ([step 1])

The resulting slurry was transferred to a 1000 L vessel polymerization reactor equipped with a stirrer for further polymerization. To the polymerization reactor, propylene was fed at 45 kg/hr, and ethylene and hydrogen were fed so that the ethylene concentration and the hydrogen concentration in the gas phase were 0.37% by mole and 0.65% by mole, respectively. The polymerization was conducted at a polymerization temperature of 72°C and at a pressure of 3.1 MPa/G.

Main polymerization-3 ([step 1])

The resulting slurry was transferred to a 500 L vessel polymerization reactor equipped with a stirrer for further polymerization. To the polymerization reactor, propylene was fed at 10 kg/hr, and ethylene and hydrogen were fed so that the ethylene concentration and the hydrogen concentration in the gas phase were 0.37% by mole and 0.65% by mole, respectively. The polymerization was conducted at a polymerization temperature of 72°C and at a pressure of 3.0 MPa/G.

Main polymerization-4 ([step 1])

The resulting slurry was transferred to a 500 L vessel polymerization reactor equipped with a stirrer for further polymerization. To the polymerization reactor, propylene was fed at 10 kg/hr, and ethylene and hydrogen were fed so that the ethylene concentration and the hydrogen concentration in the gas phase were 0.37% by mole and 0.65% by mole, respectively. The polymerization was conducted at a polymerization temperature of 72°C and at a pressure of 3.0 MPa/G.

Copolymerization ([step 2])

The resulting slurry was transferred to a 500 L vessel polymerization reactor equipped with a stirrer for copolymerization. To the polymerization reactor, propylene was fed at 10 kg/hr, and hydrogen was fed so that the hydrogen concentration in the gas phase was 0.1% by mole. Further, the polymerization was conducted by feeding ethylene to keep a polymerization temperature of 63°C and a pressure of 2.9 MPa/G.

After gasification of the resulting slurry, gas-solid separation was performed to give an ethylene-propylene random block copolymer (A2). The obtained ethylene-propylene random block copolymer (A2) was vacuum dried at 80°C.

[Production Examples 3 to 8]

Production Examples 3 to 8 were carried out in the same manner as in Production Example 2 except that the processes of main polymerization-2 and copolymerization were altered as shown in Table 1.

Table 2 shows physical property values of the ethylene-propylene block copolymers (A1) to (A8) prepared in the Production Examples. Incidentally, as compared to Production Example 2, the polymerization temperature of the copolymerization tank in Production Example 6 was decreased from 63°C to 51°C. Hence, the ethylene content in the copolymerization component was increased.

[Table 1]

Table 1

| | | Production Example 1 | Production Example 2 | Production Example 3 | Production Example 4 | Production Example 5 | Production Example 6 | Production Example 7 | Production Example 8 |
|---|---|---|---|---|---|---|---|---|---|
| Ethylene-propylene block copolymer | | A1 | A2 | A3 | A4 | A5 | A6 | A7 | A8 |
| Polymerization-2 | Ethylene conc. (mol %) | 0 | 0.37 | 0.47 | 0.37 | 1.3 | 0.37 | 0.37 | 0.37 |
| | Hydrogen conc. (mol %) | 0.45 | 0.65 | 0.5 | 0.85 | 0.65 | 0.65 | 0.65 | 0.6 |
| | Propylene feeding rate (kg/hr) | 67 | 45 | 45 | 45 | 45 | 45 | 45 | 45 |
| | Pressure (MPa/G) | 2.8 | 3.1 | 3.1 | 3.1 | 3.1 | 3.1 | 3.1 | 3.1 |
| | Polymerization temp. (°C) | 70 | 72 | 72 | 72 | 72 | 72 | 72 | 72 |
| Copolymerization | Hydrogen conc. (mol %) | 0.1 | 0.1 | 0.1 | 0.1 | 0.1 | 0.1 | 0.05 | - |
| | Polymerization temp. (°C) | 63 | 63 | 62 | 63 | 63 | 51 | 63 | - |
| | Propylene feeding rate (kg/hr) | 10 | 10 | 10 | 10 | 10 | 10 | 10 | - |
| | Pressure (MPa/G) | 2.9 | 2.9 | 2.9 | 2.9 | 2.9 | 2.9 | 2.9 | - |

[Table 2]

Table 2

| | | Production Example 1 | Production Example 2 | Production Example 3 | Production Example 4 | Production Example 5 | Production Example 6 | Production Example 7 | Production Example 8 |
|---|---|---|---|---|---|---|---|---|---|
| Ethylene-propylene block copolymer | | A1 | A2 | A3 | A4 | A5 | A6 | A7 | A8 |
| $D_{insol}$ | Ethylene amount (mol %) | 0 | 0.9 | 1.2 | 0.9 | 3.4 | 0.9 | 0.9 | 0.9 |
| | Mw/Mn | 1.9 | 2.3 | 2.3 | 2.3 | 2.3 | 2.3 | 2.3 | 2.3 |
| | 2,1-insertion propylene unit (mol %) | 0 | 0 | 0 | 0 | 0 | 0 | 0 | 0 |
| | 1,3-insertion propylene unit (mol %) | 0 | 0 | 0 | 0 | 0 | 0 | 0 | 0 |
| | Tm (°C) | 157 | 154 | 152 | 154 | 140 | 154 | 154 | 154 |
| $D_{sol}$ | Amount (wt %) | 8 | 8 | 8 | 8 | 8 | 8 | 8 | 0 |
| | Ethylene amount (mol %) | 17 | 17 | 17 | 17 | 17 | 35 | 17 | - |
| | η (dL/g) | 2.5 | 2.5 | 3 | 2.5 | 2.5 | 2.5 | 4.5 | - |
| | Mw/Mn | 2.3 | 2.3 | 2.3 | 2.3 | 2.3 | 2.3 | 2.3 | - |
| MFR (g/10 min) | | 25 | 30 | 20 | 70 | 30 | 30 | 30 | 30 |

[Example 1]

100 Parts by weight of the ethylene-propylene block copolymer (A1) was fed into a Henschel mixer, and 0.10 parts by weight of the nucleator (B), 0.10 parts by weight of the phosphorus-based antioxidant (C), 0.04 parts by weight of the amine-based antioxidant (D) and 0.03 parts by weight of the hydrochloric acid absorber (E) were added thereto, followed by stirring for mixing.

Then, the resulting mixture was melt-kneaded at a resin temperature of 200°C using a tandem extruder composed of a high performance twin screw extruder CIM50S and a single screw extruder P65EXT (65 mm φ) manufactured by The Japan Steel Works, Ltd. The kneaded product was extruded into a water tank for cooling and cutting to give pellets of a polypropylene resin.

Then, the pellets were injection molded to produce sheet-shaped test pieces having a thickness of 1 mm at a melt temperature of 200°C and a die temperature of 30°C, test pieces (ASTM D 790, thickness: 3.2 mm, length: 127 mm, width: 12.7 mm) for a bending strength test and test pieces (ASTM D 648, thickness: 6.4 mm, length: 127 mm, width: 12.7 mm) for a heat deformation temperature test at a melt temperature of 200°C and a die temperature of 40°C. These test pieces were subjected to physical property measurements according to the above-described methods. Table 3 shows the results.

[Example 2]

Example 2 was carried out in the same manner as in Example 1 except that the ethylene-propylene block copolymer (A2) was used instead of the ethylene-propylene block copolymer (A1) in Example 1. The results of the physical property measurements are shown in Table 3.

[Example 3]

Example 3 was carried out in the same manner as in Example 1 except that the ethylene-propylene block (A3) was used instead of the ethylene-propylene block copolymer (A1) in Example 1. The results of the physical property measurements are shown in Table 3.

[Comparative Example 1]

Comparative Example 1 was carried out in the same manner as in Example 1 except that the ethylene-propylene block copolymer (A4) was used instead of the ethylene-propylene block copolymer (A1) in Example 1. The results of the physical property measurements are shown in Table 3.

(Comparative Example 2]

Comparative Example 2 was carried out in the same manner as in Example 1 except that the ethylene-propylene block copolymer (A5) was used instead of the ethylene-propylene block copolymer (A1) in Example 1. The results of the physical property measurements are shown in Table 3.

(Comparative Example 3]

Comparative Example 3 was carried out in the same manner as in Example 1 except that the ethylene-propylene block copolymer (A6) was used instead of the ethylene-propylene block copolymer (A1) in Example 1. The results of the physical property measurements are shown in Table 3.

(Comparative Example 4]

Comparative Example 4 was carried out in the same manner as in Example 1 except that the ethylene-propylene block copolymer (A7) was used instead of the ethylene-propylene block copolymer (A1) in Example 1. The results of the physical property measurements are shown in Table 3.

(Comparative Example 5]

Comparative Example 5 was carried out in the same manner as in Example 1 except that the ethylene-propylene block copolymer (A8) was used instead of the ethylene-propylene block copolymer (A1) in Example 1. The results of the physical property measurements are shown in Table 3.

[Table 3]

Table 3

| | Example 1 | Example 2 | Example 3 | Comparative Example 1 | Comparative Example 2 | Comparative Example 3 | Comparative Example 4 | Comparative Example 5 |
|---|---|---|---|---|---|---|---|---|
| Ethylene-propylene block copolymer (A1) | 100 | - | - | - | - | - | - | - |
| Ethylene-propylene block copolymer (A2) | - | 100 | - | - | - | - | - | - |
| Ethylene-propylene block copolymer (A3) | - | - | 100 | - | - | - | - | - |
| Ethylene-propylene block copolymer (A4) | - | - | - | 100 | - | - | - | - |
| Ethylene-propylene block copolymer (A5) | - | - | - | - | 100 | - | - | - |
| Ethylene-propylene block copolymer (A6) | - | - | - | - | - | 100 | - | - |
| Ethylene-propylene block copolymer (A7) | - | - | - | - | - | - | 100 | - |
| Ethylene-propylene block copolymer (A8) | - | - | - | - | - | - | - | 100 |
| Nucleator (B) | 0.1 | 0.1 | 0.1 | 0.1 | 0.1 | 0.1 | 0.1 | 0.1 |
| Phosphorus-based antioxidant (C) | 0.1 | 0.1 | 0.1 | 0.1 | 0.1 | 0.1 | 0.1 | 0.1 |
| Amine-based antioxidant (D) | 0.04 | 0.04 | 0.04 | 0.04 | 0.04 | 0.04 | 0.04 | 0.04 |

(continued)

| | Example 1 | Example 2 | Example 3 | Comparative Example 1 | Comparative Example 2 | Comparative Example 3 | Comparative Example 4 | Comparative Example 5 |
|---|---|---|---|---|---|---|---|---|
| Hydrotalcite (E) | 0.03 | 0.03 | 0.03 | 0.03 | 0.03 | 0.03 | 0.03 | 0.03 |
| Bending elastic modulus (MPa) | 1500 | 1300 | 1100 | 1350 | 1000 | 1200 | 1200 | 1500 |
| Heat deformation temp. (°C) | 125 | 120 | 110 | 122 | 90 | 115 | 115 | 130 |
| Parallel optical transmittance (%) | 68 | 68 | 70 | 68 | 70 | 58 (no good) | 60 (no good) | 70 |
| Syringe breakage height (cm) | 80 | 100 | 120 | 50 (no good) | 100 | 70 | 60 | 40 (no good) |
| Syringe deformation after water vapor sterilization in elution test of the Japanese Pharmacopoeia | None (good) | None (good) | None (good) | Found (no good) | None (good) | None (good) | Found (no good) | Found (no good) |
| Syringe production cycle time (sec) | 5.3 (O) | 5.6 (O) | 5.5 (O) | Flash (no good) | 6.8 (no good) | 6.4 (no good) | 6.7 (no good) | Flash (no good) |

It is confirmed from the results of the physical property measurements shown in Table 3 that the molded products (syringes) obtained by a highly controlled polypropylene resin according to the present invention achieved well balanced transparency, shock resistance and rigidity and improvements in heat resistance and long-run moldability at levels that have not been achieved conventionally.

It has been found that all such the physical properties required as syringes can be satisfied by the ethylene-propylene block copolymer (A) constituting the polypropylene resin for syringe according to the present invention for the first time, and the industrial value of actual reduction thereof to practice is significantly high.

Furthermore, a prefilled syringe preparation comprising the syringe filled with various drug solution having a pH of 5.0 to 9.0 and achieving drug stability was obtained.

Industrial Applicability

In the case of using the polypropylene resin for syringe according to the present invention, a syringe can be produced without using other expensive auxiliary raw material, which therefore can significantly contribute to market supply of prefilled syringe preparations with low drug prices and also significantly contribute to provision of safety in medical practice.

The syringe and the prefilled syringe preparation according to the present invention can be highly expected to penetrate into market in the future and contribute to improvement in safety such as prevention of misuse or breakage of injection products and visibility of content fluid in medical practice, and are very useful.

## Claims

1. A polypropylene resin for syringe, comprising an ethylene-propylene block copolymer (A)
having a melt flow rate (ASTM D 1238, 230°C, 2.16 kg load) of 10 to 60 g/10 min,
being constituted of 85 to 97% by weight of a room temperature n-decane-insoluble portion ($D_{insol}$) and 3 to 15% by weight of a room-temperature n-decane-soluble portion ($D_{sol}$) (the total amount of the $D_{insol}$ and the $D_{sol}$ is 100% by weight), wherein
the $D_{insol}$ satisfies the following requirements (1) to (3), and the $D_{sol}$ satisfies the following requirements (5) to (7):

   (1) the melting point of the $D_{insol}$ is 150 to 165°C,
   (2) the molecular weight distribution (Mw/Mn) of the $D_{insol}$ determined by GPC is 1.0 to 3.5,
   (3) the content of the unit derived from ethylene in the $D_{insol}$ is 0 to 13% by mole,
   (5) the molecular weight distribution (Mw/Mn) of the $D_{sol}$ determined by GPC is 1.0 to 3.5,
   (6) the limiting viscosity [η] of the $D_{sol}$ in decalin at 135°C is 1.5 to 4 dl/g, and
   (7) the content of the unit derived from ethylene in the $D_{sol}$ is 15 to 25% by mole.

2. The polypropylene resin for syringe according to Claim 1, wherein the $D_{insol}$ further satisfies the following requirement (4) :

   (4) the total amount of 2,1-insertion and 1,3-insertion propylene units in the $D_{insol}$ is 0.2% by mole or less.

3. The polypropylene resin for syringe according to Claim 1 or 2, wherein the $D_{insol}$ further satisfies the following requirement (1'):

   (1') the melting point of the $D_{insol}$ is 150 to 160°C.

4. The polypropylene resin for syringe according to any one of Claims 1 to 3, wherein the $D_{insol}$ further satisfies the following requirement (3'):

   (3') the content of the unit derived from ethylene in the $D_{insol}$ is 0 to 4% by mole.

5. A syringe in which a tubular part (barrel) obtained by injection molding of the polypropylene resin for syringe according to any one of Claims 1 to 4 is equipped with a piston (plunger).

6. A prefilled syringe preparation comprising the syringe according to Claim 5 filled with a drug solution having a pH of 5.0 to 9.0.

## Patentansprüche

1. Polypropylenharz für eine Spritze, umfassend ein Ethylenpropylenblockcopolymer (A) mit einer Schmelzflussrate (ASTM D 1238, 230°C, 2,16 kg Beladung) von 10 bis 60 g/10 min, das aus 85 bis 97 Gew.-% eines bei Raumtemperatur in n-Dekan-unlöslichen Teils ($D_{unlös}$) und 3 bis 50 Gew.-% eines bei Raumtemperatur in n-Dekan-löslichen Teils ($D_{lös}$) gebildet ist (wobei die Gesamtmenge aus $D_{unlös}$ und aus $D_{lös}$ 100 Gew.-% beträgt), wobei $D_{unlös}$ den folgenden Erfordernissen (1) bis (3) genügt und $D_{lös}$ den folgenden Erfordernissen (5) bis (7) genügt:

    (1) der Schmelzpunkt von $D_{unlös}$ beträgt 150 bis 165°C,
    (2) die durch GPC bestimmte Molekulargewichtsverteilung (Mw/Mn) von $D_{unlös}$ beträgt 1,0 bis 3,5,
    (3) der Gehalt der aus Ethylen abgeleiteten Einheit in $D_{unlös}$ beträgt 0 bis 13 mol-%,
    (5) die durch GPC bestimmte Molekulargewichtsverteilung (Mw/Mn) von $D_{lös}$ beträgt 1,0 bis 3,5,
    (6) die Grenzviskosität [η] von $D_{lös}$ in Decalin bei 135°C beträgt 1,5 bis 4 dl/g, und
    (7) der Gehalt der aus Ethylen abgeleiteten Einheit in $D_{lös}$ beträgt 15 bis 25 mol-%.

2. Polypropylenharz für eine Spritze nach Anspruch 1, wobei $D_{unlös}$ ferner dem folgenden Erfordernis (4) genügt:

    (4) die Gesamtmenge an 2,1-Insertions- und 1,3-Insertions-Propyleneinheiten in $D_{unlös}$ beträgt 0,2 mol-% oder weniger.

3. Polypropylenharz für eine Spritze nach Anspruch 1 oder 2, wobei $D_{unlös}$ ferner dem folgenden Erfordernis (1') genügt:

    (1') der Schmelzpunkt von $D_{unlös}$ beträgt 150 bis 160°C.

4. Polypropylenharz für eine Spritze nach einem der Ansprüche 1 bis 3, wobei $D_{unlös}$ ferner dem folgenden Erfordernis (3') genügt:

    (3') der Gehalt der aus Ethylen abgeleiteten Einheit in $D_{unlös}$ beträgt 0 bis 4 mol-%.

5. Spritze, in der ein rohrförmiger Teil (Zylinder), der durch Spritzgießen des Polypropylenharzes für eine Spritze nach einem der Ansprüche 1 bis 4 erhalten ist, mit einem Kolben (Stößel) ausgestattet ist.

6. Vorgefüllte Spritzenpräparation, umfassend die Spritze nach Anspruch 5, die mit einer Wirkstofflösung mit einem pH von 5,0 bis 9,0 gefüllt ist.

## Revendications

1. Résine de polypropylène pour seringue, comprenant un copolymère à blocs (A) d'éthylène et de propylène

    - qui présente un indice de fluidité à chaud (norme ASTM D-1238, 230 °C, charge de 2,16 kg) de 10 à 60 g/10 min,
    - et qui est constitué de 85 à 97 % en poids d'une fraction insoluble dans du n-décane à température ambiante ($D_{insol}$) et de 3 à 15 % en poids d'une fraction soluble dans du n-décane à température ambiante ($D_{sol}$), étant entendu que le total des fractions $D_{insol}$ et $D_{sol}$ fait 100 % en poids,

    laquelle fraction $D_{insol}$ respecte les conditions (1) à (3) suivantes, et laquelle fraction $D_{sol}$ respecte les conditions (5) à (7) suivantes :

    1) le point de fusion de la fraction $D_{insol}$ vaut de 150 à 165°C ;
    2) l'indice Mw/Mn de distribution des masses moléculaires de la fraction $D_{insol}$, déterminé par GPC, vaut de 1,0 à 3,5 ;
    3) la proportion de motifs dérivés de l'éthylène dans la fraction $D_{insol}$ vaut de 0 à 13 % en moles ;
    5) l'indice Mw/Mn de distribution des masses moléculaires de la fraction $D_{sol}$, déterminé par GPC, vaut de 1,0 à 3,5 ;
    6) l'indice limite de viscosité [η] de la fraction $D_{sol}$, mesurée dans de la décaline à 135 °C, vaut de 1,5 à 4 dL/g ;
    7) et la proportion de motifs dérivés de l'éthylène dans la fraction $D_{sol}$ vaut de 15 à 25 % en moles.

**2.** Résine de polypropylène pour seringue, conforme à la revendication 1, dans laquelle la fraction $D_{insol}$ respecte en outre la condition (4) suivante :

4) la proportion totale de motifs de propylène insérés en mode 2,1 et en mode 1,3 dans la fraction $D_{insol}$ est inférieure ou égale à 0,2 % en moles.

**3.** Résine de polypropylène pour seringue, conforme à la revendication 1 ou 2, dans laquelle la fraction $D_{insol}$ respecte en outre la condition (1') suivante :

1') le point de fusion de la fraction $D_{insol}$ vaut de 150 à 160 °C.

**4.** Résine de polypropylène pour seringue, conforme à l'une des revendications 1 à 3, dans laquelle la fraction $D_{insol}$ respecte en outre la condition (3') suivante :

3') la teneur en motifs dérivés de l'éthylène de la fraction $D_{insol}$ vaut de 0 à 4 % en moles.

**5.** Seringue dont la partie tubulaire (corps) a été obtenue par moulage par injection d'une résine de polypropylène pour seringue, conforme à l'une des revendications 1 à 4, et est équipée d'un piston (poussoir).

**6.** Préparation en seringue pré-remplie, comprenant une seringue conforme à la revendication 5, remplie d'une solution de médicament présentant un pH de 5,0 à 9,0.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3195434 B
- JP 2528443 B
- JP H06192332 B
- WO 0127124 A
- JP H11315109 B
- JP H07145212 B
- WO 2006068308 A


### Non-patent literature cited in the description


- Test Methods for Plastic Containers for Pharmaceutical Products Test. General Tests of the Japanese Pharmacopoeia
- General Tests of the Japanese Pharmacopoeia